(19) Europäisches Patentamt
European Patent Office
Office européen des brevets

(11) EP 2 364 141 B2

(12) # NEUE EUROPÄISCHE PATENTSCHRIFT

Nach dem Einspruchsverfahren

(45) Veröffentlichungstag und Bekanntmachung des Hinweises auf die Entscheidung über den Einspruch:
**02.08.2017 Patentblatt 2017/31**

(45) Hinweis auf die Patenterteilung:
**05.03.2014 Patentblatt 2014/10**

(21) Anmeldenummer: **09765042.8**

(22) Anmeldetag: **04.12.2009**

(51) Int Cl.:
***A61K 9/16*** *(2006.01)* ***A61K 31/496*** *(2006.01)*

(86) Internationale Anmeldenummer:
**PCT/EP2009/008692**

(87) Internationale Veröffentlichungsnummer:
**WO 2010/066385 (17.06.2010 Gazette 2010/24)**

(54) **KOMPAKTIERTES MOXIFLOXACIN**

COMPACTED MOXIFLOXACIN

MOXIFLOXACINE COMPACTÉE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **08.12.2008 DE 102008061083**

(43) Veröffentlichungstag der Anmeldung:
**14.09.2011 Patentblatt 2011/37**

(60) Teilanmeldung:
**14155501.1 / 2 735 307**
**14193132.9 / 2 837 376**

(73) Patentinhaber: **ratiopharm GmbH**
**89079 Ulm (DE)**

(72) Erfinder:
- **SWATSCHEK, Dieter**
**89143 Blaubeuren (DE)**
- **SCHEIWE, Max, Werner**
**79689 Maulburg (DE)**

(74) Vertreter: **Ter Meer Steinmeister & Partner**
**Patentanwälte mbB**
**Nymphenburger Straße 4**
**80335 München (DE)**

(56) Entgegenhaltungen:
**WO-A1-03/103674 WO-A1-2004/091619**
**WO-A1-2007/128349 WO-A2-2009/135646**

EP 2 364 141 B2

## Beschreibung

Die Erfindung betrifft ein Verfahren zur Herstellung von Tabletten enthaltend Moxifloxacin, umfassend die Schritte (i) Bereitstellen von Moxifloxacin pharmazeutisch verträglicher Salze, Hydrate oder Solvate davon im Gemisch mit einem Adhäsionsmittel; (ii) Kompaktierung zu einer Schülpe; (iii) Granulierung der Schülpe; und (iv) Kompression der resultierenden Granulate zu Tabletten; sowie Tabletten, Granulate und Schülpen enthaltend kompaktiertes Moxifloxacin. Ferner betrifft die Offenbarung Tabletten enthaltend Moxifloxacin mit einer bimodalen Porengrößenverteilung.

1-Cyclopropyl-7-((S,S)-2,8-diazabicyclo[4.3.0]non-8-yl)-6-fluor-1,4-dihydro-8-methoxy-4-oxo-3-chinoloncarbonsäure ist unter dem INN-Namen "Moxifloxacin" bekannt und weist folgende Strukturformel auf:

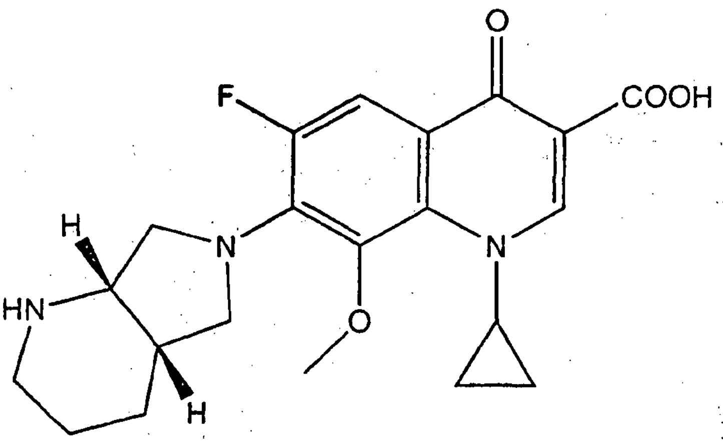

Moxifloxacin

Moxifloxacin ist ein Antibiotikum, das zur Therapie von Atemwegsinfektionen und einer Pneumonie verwendet wird. Moxifloxacin wirkt bakterizid durch die Hemmung der bakteriellen DNS-Topoisomerasen II (DNS-Gyrase) und IV, die für die DNS-Replikation und Transkription in der Bakterienzelle verantwortlich sind.

Die Synthese von Moxifloxacin ist in EP 0 350 733 A2, EP 0 757 990 B1, beziehungsweise DE 42 00 414 A1 beschrieben.

In EP 0 780 390 A1 wurde gefunden, dass vorteilhafte pharmazeutische Formulierungen dann erhalten werden, wenn Moxifloxacin in Form des Monohydrats verwendet wird. Des Weiteren wurde ein Verfahren bereitgestellt, bei dem kristallines Moxifloxacin nicht in Form von Nadeln anfällt, denn Nadeln würden unerwünscht verfilzen und die Rieselfähigkeit des Wirkstoffes beeinträchtigen.

EP 1 017 392 B1 beschreibt Moxifloxacin-Arzneimittelzubereitungen mit kontrollierter Wirkstofffreisetzung. Die verzögerte Freisetzung wurde durch Zugabe eines Diffusionslackes erreicht. Die Herstellung dieser Formulierungen erfolgte durch Nassgranulierung in der Wirbelschicht.

WO 2005/20998 A1 beschreibt ebenfalls Moxifloxacin-Formulierungen, wobei ein wasserunlöslicher Hilfsstoff mittels Feuchtgranulation intragranulär und extragranulär verarbeitet wird. Ziel dieser Herstellungsmethode war es, eine bioäquivalente Formulierung zu Avelox® bereit zu stellen.

WO 2007/128349 A1 offenbart pharmazeutische Zusammensetzungen, die einerseits auf pyrogener Kieselsäure basierende Schülpen und andererseits mindestens einen pharmazeutisch aktiven Bestandteil enthalten. Diese pharmazeutischen Zusammensetzungen können außerdem noch einen pharmazeutischen Hilfsstoff enthalten. Ferner wird die Verwendung von auf pyrogener Kieselsäure basierenden Schülpen in einer pharmazeutischen Zusammensetzung offenbart.

Schließlich wurde in EP 1 128 831 A gefunden, dass Tabletten mit hoher Bruchlast bzw. Härte nur dann erhalten werden, wenn Lactose als Hilfsstoff verwendet wird und der Lactosegehalt der Formulierung zwischen 2,5 und 25 % liegt. Die Verwendung von Lactose ist jedoch oftmals unerwünscht, da ein nicht unerheblicher Anteil der Patienten an Lactoseunverträglichkeit leidet.

Es lässt sich folglich feststellen, dass die im Stand der Technik beschriebenen Verfahren zur Herstellung von Moxifloxacin enthaltenden Tabletten zahlreiche Nachteile aufweisen, beispielsweise durch spezielle Anforderungen an den Lactosegehalt, an die Kristallstruktur des Wirkstoffs, beziehungsweise durch die Notwendigkeit von speziellen Lösungen zur Granulierung. Ferner sind die bekannten Tabletten nur bedingt zur Lackierung geeignet. Aufgabe der Erfindung war es daher, die im Stand der Technik auftretenden Nachteile zu überwinden. Insbesondere sollen die Nachteile des Standes der Technik ohne Beeinträchtigung der Arbeitssicherheit überwunden werden.

Im Speziellen war es Aufgabe der Erfindung ein Verfahren zur Herstellung von Moxifloxacin enthaltenden Tabletten bereit zu stellen, wobei Moxifloxacin mit jedem bekannten Kristallhabitus (z.B. Plättchen oder Nadeln) und/oder

in jeder der bekannten polymorphen Formen verarbeitet werden kann, d.h. es soll eine Verarbeitung von Moxifloxacin mit unterschiedlicher Wirkstoffbeschaffenheit ermöglicht werden. Ebenfalls soll eine Verarbeitung von Moxifloxacin mit variablem Wassergehalt ermöglicht werden. Die Verarbeitung von Moxifloxacin mit variablem Wassergehalt soll vor allem bei zeitgleich vorteilhafter Lagerstabilität ermöglicht werden.

Im Speziellen war es Aufgabe der Erfindung Tabletten bereit zu stellen, die sowohl eine rasche Zerfallszeit (weniger als 15 Minuten, bevorzugt weniger als 10 Minuten) als auch eine möglichst vorteilhafte Bruchfestigkeit (mehr als 160 Newton, bevorzugt mehr als 180 Newton) aufweisen. Zudem sollen die resultierenden Tabletten geringen Abrieb zeigen.

Insbesondere war es Aufgabe der Erfindung, ein Verfahren zur Herstellung von Moxifloxacin enthaltenden Tabletten bereit zu stellen, die eine vorteilhafte Lackierbarkeit zeigen. Bei der Lackierung der erfindungsgemäßen Tabletten soll es zu keinen "Abplatzern" kommen.

Ebenfalls sollte eine Granulatformulierung von Moxifloxacin bereitgestellt werden, die vorteilhaft zur Herstellung einer Suspension zum Einnehmen verwendet werden kann. Die Granulate sollen gut fließen, sich während der Lagerung nicht entmischen und eine exakte Dosierung aus Ein- und Mehrdosenbehältnissen ermöglichen.

Schließlich war es Aufgabe der Erfindung, pharmazeutische Darreichungsformen von Moxifloxacin bereit zu stellen, die eine vorteilhafte Lagerstabilität aufweisen. Ebenfalls soll eine vorteilhafte Gleichförmigkeit des Gehalts (*content uniformity*) gewährleistet sein. Alle der vorstehend genannten Aufgaben sollen insbesondere für einen hohen Wirkstoffgehalt (drug *load*) gelöst werden. Ferner sollen die Aufgaben unter Vermeidung von Lactose als Hilfsstoff gelöst werden können.

Die Erfinder haben nun unerwartet gefunden, dass die Aufgaben durch Kompaktierung eines Gemisches aus Moxifloxacin und Adhäsionsmittel zu einer Schülpe gelöst werden konnten.

Gegenstand der Erfindung ist daher eine Schülpe enthaltend Moxifloxacin, erhältlich durch ein Verfahren umfassend die Schritte

(i) Bereitstellen von Moxifloxacin oder pharmazeutisch verträglicher Salze davon im Gemisch mit einem Adhäsionsmittel; und
(ii) Kompaktierung zu einer Schülpe; wobei die Kompaktierung in einem Walzengranulator durchgeführt wird und wobei die Spaltbreite des Walzengranulators 2 bis 4 mm und wobei die Walzkraft 2 bis 30 kN/cm beträgt.

Gegenstand der Erfindung ist ferner ein Verfahren zur Herstellung von Tabletten enthaltend Moxifloxacin, umfassend die Schritte

(i) Bereitstellen von Moxifloxacin oder pharmazeutisch verträglicher Salze davon im Gemisch mit einem Adhäsionsmittel;

(ii) Kompaktierung zu einer Schülpe; wobei die Kompaktierung in einem Walzengranulator durchgeführt wird und wobei die Spaltbreite des Walzengranulators 2 bis 4 mm und wobei die Walzkraft 2 bis 30 kN/cm beträgt.

(iii) Granulierung der Schülpe; und

(iv) Kompression der resultierenden Granulate zu Tabletten, gegebenenfalls unter Zusatz weiterer pharmazeutischer Hilfsstoffe.

Die mit dem erfindungsgemäßen Verfahren hergestellten Tabletten können gegebenenfalls in einem weiteren, optionalen Schritt (v) befilmt werden.

Ebenfalls sind Tabletten und befilmte Tabletten, erhältlich durch das erfindungsgemäße Verfahren, Gegenstand dieser Erfindung.

Weiterhin ist Gegenstand der Erfindung ein Granulat, insbesondere zur Befüllung von Sachets oder Kapseln, enthaltend Moxifloxacin, erhältlich durch ein Verfahren umfassend die Schritte

(i) Bereitstellen von Moxifloxacin oder pharmazeutisch verträglicher Salze davon im Gemisch mit einem Adhäsionsmittel;

(ii) Kompaktierung zu einer Schülpe; wobei die Kompaktierung in einem Walzengranulator durchgeführt wird und wobei die Spaltbreite des Walzengranulators 2 bis 4 mm und wobei die Walzkraft 2 bis 30 kN/cm beträgt, und

(iii) Granulierung der Schülpe.

Während oder bevorzugt nach dem Schritt (iii) können optional weitere Hilfsstoffe dem Granulat zugefügt werden. Insbesondere werden Hilfsstoffe zur Verbesserung der Fließfähigkeit, Klebeneigung, Zerfallseigenschaften, Geschmack und/oder Benetzbarkeit hierfür verwendet.

Das resultierende Granulat wird bevorzugt zur Herstellung einer Suspension zum Einnehmen verwendet. Es wird bevorzugt in ein geeignetes Packmittel gefüllt. Beispiele für Packmittel sind Kapseln. Flaschen, Dosen oder bevorzugt Sachets. Im Falle von Flaschen oder Dosen können diese eine Tagesdosis beinhalten. Alternativ können auch Mehrtagesdosen, z.B. eine 10-Tagesdosis, in Flaschen oder Dosen gefüllt werden.

Schließlich ist Gegenstand der Offenbarung die Verwendung von trockenkompaktiertem Moxifloxacin zur oralen Therapie von Infektionen, insbesondere von Infektionen der Atemwege und Weichteil-Infektionen.

Das erfindungsgemäße Verfahren zur Herstellung von Tabletten enthaltend Moxifloxacin wird in den nachstehenden Absätzen ausführlich erläutert.

In Schritt (i) des erfindungsgemäßen Verfahrens wird zunächst Moxifloxacin bereitgestellt.

Grundsätzlich umfasst im Rahmen dieser Anmeldung der Begriff "Moxifloxacin" sowohl Moxifloxacin in Form der freien Base als auch pharmazeutisch verträgliche Salze davon. Hierbei kann es sich um ein oder mehrere Salze handeln, die auch im Gemisch vorliegen können. Weiterhin umfasst der Begriff "Moxifloxacin" auch mögliche Hydrate oder Solvate.

Bevorzugt werden als Salze Säureadditionssalze verwendet. Beispiele für geeignete Salze sind Hydrochloride, Carbonate, Hydrogencarbonate, Acetate, Lactate, Butyrate, Propionate, Sulfate, Citrate, Tartrate, Nitrate, Sulfonate, Oxalate und/oder Succinate. Im Rahmen dieser Erfindung wird Moxifloxacin bevorzugt in Form der freien Base oder als Moxifloxacin-Hydrochlorid eingesetzt.

Das verwendete Moxifloxacin kann Wasser enthalten. Üblicherweise umfasst es 0.1 bis 5 Gew.-% Wasser, bevorzugt 0,2 bis 2 Gew.-% Wasser, bezogen auf das Gesamtgewicht des Wirkstoffs.

Im Schritt (i) des erfindungsgemäßen Verfahrens wird Moxifloxacin mit einem oder mehreren Adhäsionsmittel(n) vermischt.

Unter "Adhäsionsmittel" sind im Allgemeinen Mittel zu verstehen, welche die Adhäsionseigenschaften des resultierenden Kompaktats verbessern. Weiterhin zeichnen sich Adhäsionsmittel bevorzugt dadurch aus, dass Sie die Plastizität der Tablettiermischung erhöhen, so dass bei der Verpressung feste Tabletten entstehen.

In einer möglichen Ausführungsform handelt es sich bei dem Adhäsionsmittel um ein Polymer. Ferner umfasst der Begriff "Adhäsionsmittel" auch Stoffe, die sich polymerähnlich verhalten. Beispiele hierfür sind Fette und Wachse. Weiterhin umfasst das Adhäsionsmittel feste, nicht-polymere Verbindungen, die bevorzugt polare Seitengruppen aufweisen. Beispiele hierfür sind Zuckeralkohole oder Disaccharide.

Ferner weist das als Adhäsionsmittel verwendbare Polymer bevorzugt ein zahlenmittleres Molekulargewicht von 1.000 bis 500.000 g/mol, mehr bevorzugt von 2.000 bis 90.000 g/mol, auf. Wird das zur Herstellung des Intermediats verwendete Polymer in Wasser in einer Menge von 2 Gew.-% gelöst, so zeigt die resultierende Lösung bevorzugt eine Viskosität von 0,1 bis 8 mPa/s, mehr bevorzugt von 0,3 bis 7 mPa/s, insbesondere von 0,5 bis 4 mPa/s, gemessen bei 25 °C. Die Viskosität wird bevorzugt gemäß Ph. Eur. 6.0, Abschnitt 2.2.10. bestimmt.

Bevorzugt werden zur Herstellung des Intermediats hydrophile Polymere verwendet. Darunter sind Polymere zu verstehen, die hydrophile Gruppen aufweisen. Beispiele für geeignete hydrophile Gruppen sind Hydroxy, Alkoxy, Acrylat, Methacrylat. Sulfonat, Carboxylat und quartäre Ammoniumgruppen.

Das erfindungsgemäße Intermediat kann beispielsweise folgende Polymere als Adhäsionsmittel umfassen: Polysaccharide, wie Hydroxypropylmethylcellulose (HPMC), Carboxymethylcellulose (CMC, insbesondere Natrium- und Calciumsalze), Ethylcellulose, Methylcellulose, Hydroxyethylcellulose, Ethylhydroxyethylcellulose, Hydroxypropylcellulose (HPC); mikrokristalline Cellulose, Guar-Mehl, Alginsäure und/oder Alginate; synthetische Polymere wie Polyvinylpyrrolidon, Polyvinylacetat (PVAC), Polyvinylalkohol (PVA), Polymere der Acrylsäure und deren Salze, Polyacrylamid, Polymethacrylate, Vinylpyrrolidon-Vinylacetat-Copolymere (beispiels-weise Kollidon® VA64, BASF), Polyalkylenglykole, wie Polypropylenglykol oder bevorzugt Polyethylenglykol, Co-blockpolymere des Polyethylenglykols, insbesondere Co-blockpolymere aus Polyethylenglykol und Polypropylenglykol (Pluronic®, BASF) sowie Gemische aus den genannten Polymeren.

Bei dem im Rahmen dieser Erfindung verwendeten Adhäsionsmittel kann es sich um ein Polymer handeln, das eine Glasübergangstemperatur (Tg) von größer 15 °C, mehr bevorzugt von 40 °C bis 150 °C, insbesondere von 50 °C bis 110 °C, aufweist.

Als "Glasübergangstemperatur" (Tg) bezeichnet man die Temperatur, bei der amorphe oder teilkristalline Polymere vom festen Zustand in den flüssigen Zustand übergehen. Dabei tritt eine deutliche Änderung physikalischer Kenngrößen, z. B. der Härte und der Elastizität, ein. Unterhalb der Tg ist ein Polymer üblicherweise glasartig und hart, oberhalb der Tg geht es in einen gummiartigen bis zähflüssigen Zustand über. Die Bestimmung der Glasübergangstemperatur erfolgt im Rahmen dieser Erfindung mittels dynamischer Differenzkalorimetrie (DSC). Hierzu kann z. B. ein Gerät von Mettler Toledo DSC 1 eingesetzt werden. Es wird mit einer Heizrate von 1-20 °C/min, bevorzugt 5-15 °C/min, bzw. mit einer Kühlrate von 5-25, bevorzugt 10 -20 °C/min, gearbeitet.

Ferner umfasst das Adhäsionsmittel auch feste, nicht-polymere Verbindungen, die bevorzugt polare Seitengruppen aufweisen. Beispiele hierfür sind Zuckeralkohole oder Disaccharide. Beispiele für geeignete Zuckeralkohole und/oder Disaccharide sind Mannitol, Sorbitol, Xylitol, Glucose, Fructose, Maltose und Gemische daraus. Der Begriff Zuckeralkohole umfasst hier auch Monosaccharide. Insbesondere wird Sorbitol und/oder Mannitol als Adhäsionsmittel verwendet.

Weiterhin sind Gemische der genannten Adhäsionsmittel möglich.

In einer besonders bevorzugten Ausführungsform handelt es sich bei dem Adhäsionsmittel um ein Mittel enthaltend oder bestehend aus mikrokristalliner Cellulose. In einer alternativen bevorzugten Ausführungsform handelt es sich bei dem Adhäsionsmittel um ein Mittel enthaltend oder bestehend aus mikrokristalliner Cellulose und Zuckeralkohol, insbesondere um ein Mittel enthaltend oder bestehend aus mikrokristalliner Cellulose sowie Sorbitol und/oder Mannitol. Das Gewichtsverhältnis mikrokristalline Cellulose zu Zuckeralkohol beträgt hierbei 1 : 5 bis 5 : 1, bevorzugt von 1 : 1 bis 3 : 1.

In bevorzugten Ausführungsformen der vorliegenden Erfindung werden Moxifloxacin und Adhäsionsmittel in einer Menge verwendet, wobei das Gewichtsverhältnis von Moxifloxacin zu Adhäsionsmittel 10 : 1 bis 1 : 10, mehr bevorzugt 5 : 1 bis 1 : 3, noch mehr bevorzugt 3 : 1 bis 1 : 2, insbesondere 2,5 : 1 bis 1,5 : 1, beträgt.

Es ist vorteilhaft, wenn das Adhäsionsmittel in partikulärer Form eingesetzt wird und das Adhäsionsmittel eine volumerimittlere Teilchengröße (D50) weniger als 500 $\mu$m, bevorzugt 5 bis $\mu$m, beträgt:

Der Ausdruck "mittlerer Teilchendurchmesser" oder "volumenmittlere Teilchengröße" bezieht sich im Rahmen dieser Erfindung auf den D50-Wert des volumenmittleren Teilchendurchmessers, der mittels Laserdiffraktometrie bestimmt wurde. Insbesondere wurde zur Bestimmung ein Mastersizer 2000 von Malvern Instruments verwendet (Nassmessung (bevorzugt Paraffin als Dispergiermittel), mit Ultraschall 60 sek., 2000 UpM, wobei die Auswertung nach dem Fraunhofer Modell erfolgt). Der mittlere Teilchendurchmesser, der auch als D50-Wert der integralen Volumenverteilung bezeichnet wird, wird im Rahmen dieser Erfindung als der Teilchendurchmesser definiert. bei dem 50 Volumen-% der Teilchen einen kleineren Durchmesser haben als der Durchmesser, der dem D50-Wert entspricht. Ebenso haben dann 50 Volumen-% der Teilchen einen größeren Durchmesser als der D50-Wert. Analog wird der D90-Wert der integralen Volumenverteilung als der Teilchendurchmesser definiert, bei dem 90 Volumen-% der Teilchen einen kleineren Durchmesser haben als der Durchmesser, der dem D90-Wert entspricht.

Im Schritt (i) des erfindungsgemäßen Verfahrens können dem Gemisch aus Moxifloxacin und Adhäsionsmittel gegebenenfalls noch weitere pharmazeutische Hilfsstoffe zugesetzt werden. Hierbei handelt es sich bevorzugt um die nachstehend (unter Verfahrensschritt (iv)) näher beschriebenen Hilfsstoffe. Grundsätzlich ist es in diesem Zusammenhang möglich, dass die beschriebenen Hilfsstoffe im Schritt (i), im Schritt (iv) oder teilweise im Schritt (i) und Schritt (iv) zugesetzt werden. In einer bevorzugten Ausführungsform wird im Schritt (i) Sprengmittel und im Schritt (iv) Fließregulierungsmittel und/oder Schmiermittel zugesetzt.

In einer bevorzugten Ausführungsform werden in Schritt (1) des erfindungsgemäßen Verfahrens

(a) 20 bis 80 Gew.-%, mehr bevorzugt 40 bis 75 Gew.-%, insbesondere 55 bis 70 Gew. % Moxifloxacin oder pharmazeutisch verträgliche Salze davon und
(b) 20 bis 80 Gew.-%, mehr bevorzugt 25 bis 60 Gew.-%, insbesondere 45 bis 30 Gew.-% Adhäsionsmittel,

bezogen auf das Gesamtgewicht der eingesetzten Mischung, vermischt. Sofern zusammen mit dem Adhäsionsmittel weitere pharmazeutische Hilfsstoffe eingesetzt werden, beziehen sich vorstehend gemachte Angaben der Komponente (b) auf das Gewicht von Adhäsionsmittel und weiteren pharmazeutischen Hilfsstoffen. In einer bevorzugten Ausführungsform bezieht sich die Angabe der Komponente (b) auf das Gewicht von Adhäsionsmittel und Sprengmittel.

Die Vermischung kann in üblichen Mischern erfolgen. Beispielsweise kann die Vermischung in Zwangsmischern oder Freifallmischern erfolgen, z.B. mittels Turbula T 10B (Bachofen AG, Schweiz). Alternativ ist es möglich, dass das Moxifloxacin zunächst nur mit einem Teil der Adhäsionsmittel (z.B. 50 bis 95 Gew.-%) vor der Kompaktierung (b) vermischt wird, und dass der verbleibende Teil der Hilfsstoffe nach dem Granulierschritt (c) zugegeben wird. Im Falle der Mehrfachkompaktierung sollte das Zumischen der Hilfsstoffe bevorzugt vor dem ersten Kompaktierschritt, zwischen mehreren Kompaktierschritten oder nach dem letzten Granulierschritt erfolgen.

Das in Schritt (i) eingesetzte Moxifloxacin kann eine volumenmittlere Teilchengröße (D50) von beispielsweise mehr als 20 bis 200 $\mu$m, bevorzugt von 50 bis 150 $\mu$m, aufweisen.

Das eingesetzte Moxifloxacin kann alternativ mikronisiert werden. Die Mikronisierung erfolgt bevorzugt vor der Kompaktierung bzw. vor dem Vermischen des Moxifloxacins mit den Hilfsstoffen. Die Mikronisierung führt üblicherweise zu einer Erhöhung der Oberflächenrauhigkeit. Die Mikronisierung erfolgt beispielsweise in Stiftmühlen oder Luftprallmühlen. Die Mikronisierung kann auch durch Nassmahlen in Kugelmühlen erfolgen. Das mikronisierte Moxifloxacin weist bevorzugt eine volumenmittlere Teilchengröße (D(50)) von 0,5 bis 20 $\mu$m, bevorzugt von 1 bis 10 $\mu$m auf.

Im Schritt (ii) des erfindungsgemäßen Verfahrens wird das Gemisch, enthaltend Moxifloxacin und Adhäsionsmittel (und gegebenenfalls weitere pharmazeutische Hilfsstoffe) aus Schritt (i), zur erfindungsgemäßen Schülpe kompaktiert. Hierbei handelt es sich um eine Trockenkompaktierung. Anstelle des Begriffes "Schülpe" wird daher im Rahmen dieser Erfindung auch der Ausdruck "Kompaktat" verwendet.

Die Kompaktierung erfolgt in Abwesenheit von Lösungsmitteln, insbesondere in Abwesenheit von organischen Lösungsmitteln.

Die Kompaktierung wird in einem Walzengranulator durchgeführt.

Die Walzkraft beträgt 2 bis 30 kN/cm, bevorzugt 5 bis 15 kN/cm, insbesondere 6 bis 12 kN/cm.

Die Spaltbreite des Walzgranulators beträgt 2 bis 4 mm, bevorzugt 3,0 bis 4,0 mm, insbesondere 3,2 bis 3,8 mm.

Die verwendete Kompaktiervorrichtung weist bevorzugt eine Kühlvorrichtung auf. Insbesondere wird der Prozess so geführt und gegebenenfalls gekühlt, so dass die Temperatur des Kompaktats 55 °C nicht überschreitet.

Der typische Durchsatz durch den Kompaktor beträgt üblicherweise 12 - 45 kg/h, bevorzugt 15 - 30 kg/h. Hierfür wird bevorzugt die oben genannte Spaltenbreite und eine Walzenbreite von 100 mm verwendet.

Die Mischbedingungen in Schritt (i) und/oder die Kompaktierbedingungen in Schritt (ii) werden üblicherweise so gewählt, dass mindestens 10 % der Oberfläche der resultierenden Moxifloxacin-Teilchen mit Adhäsionsmittel bedeckt sind, mehr bevorzugt mindestens 20 % der Oberfläche, besonders bevorzugt mindestens 50 % der Oberfläche, insbesondere mindestens 60 % der Oberfläche.

Die Kompaktierungsbedingungen im Schritt (ii) werden im Allgemeinen so gewählt, dass die Schülpe eine scheinbare Dichte von 0,8 bis 1,3 g/cm$^3$, bevorzugt von 0,86 bis 1,28 g/cm$^3$, mehr bevorzugt von 0.92 bis 1.21 g/cm$^3$, insbesondere von 1,00 bis 1,18 g/cm$^3$ aufweist.

Die scheinbare Dichte der Schülpe berechnet sich wie folgt:

$$\text{scheinbare Dichte}_{\text{Schülpe}} = \text{Masse}_{\text{Schülpe}} / \text{Volumen}_{\text{Schülpe}}$$

Im Allgemeinen eignen sich zwei Methoden zur Bestimmung der scheinbaren Dichte, nämlich a) Durchsatzmethode und b) Stanzmethode.

a) Durchsatzmethode:

Die Durchsatzmethode ist insbesondere bei der Verwendung eines Walzenkompaktors oder Walzengranulators bevorzugt.

Ermittlung der Durchsatzrate beim Trockenkompaktieren:

Die Messung erfolgt nach dem Wägeprinzip, indem die kompaktierte Masse (= Schülpe aus Schritt (ii)) in einem definierten Zeitraum unter ansonsten konstanten Bedingungen aufgefangen und genau verwogen wird. Eine eventuelle Feuchtezunahme des Kompaktats wird anschließend rechnerisch korrigiert.

Dazu wird - nachdem der Kompaktor mit konstanter Walzendrehzahl, Spaltweite und Kompaktierkraft arbeitet, d.h. die Anlaufphase in die Produktionsphase übergangen ist - das Kompaktat in einem pharmagerechten Behälter restlos und verlustfrei aufgefangen und die zugehörige Prozesszeit wird erfasst. Hierfür, wird mit der Stoppuhr ein Zeitraum von 2 Minuten entsprechend 120 sec festgelegt, und das in dieser Zeit aufgefangene Kompaktat wird für die Messung verwendet. Danach wird das Kompaktat verwogen, und die Feuchte bestimmt (Halogenlampenfeuchtemesser). Die gewogene Masse wird mit der Feuchtedifferenz vor und nach Kompaktieren korrigiert, und dann der Massenfluss errechnet durch Teilen der Masse in kg durch die Zeit in Minuten.

Ergebnis: Durchsatz Kompaktat in kg/min. Durch Multiplizieren mit 60 erhält man den Durchsatz Kompaktat in kg/h.

Messprinzip eines Halogenlampenfeuchtemessers:

Die Messmethode ist Thermogravimetrie, d.h. eine definierte Masse wird thermisch angeregt und gibt gegebenenfalls Wasser ab. Die Gewichtsveränderung ist ein Maß für die vorhandene Feuchte (siehe z.B. Mettler-Toledo: Halogen Moisture Analyzer HG 63).

Die Berechnung der scheinbaren Dichte erfolgt dann durch folgende Formel:

$$\text{scheinbare Dichte} = \text{Massendurchsatz} / \text{Volumendurchsatz;}$$

wobei

**Volumendurchsatz = Walzendrehzahl x Walzenbreite x Walzendurchmesser x Kreiszahl $\pi$ (pi) x Spaltbreite.**

b) Stanzmethode:

Die Stanzmethode beruht auf dem Prinzip der Isolierung und Verwiegung eines geometrisch definierten Probenkörpers aus der Schülpe.

Mit Hilfe eines Stanzmessers wird ein definiertes Schülpenstück aus der Probe separiert. Das Volumen dieses Schülpenstücks entspricht dem Stanzvolumen.

Es wird ein zylindrisches Stanzstück verwendet. Das Volumen des Stanzstücks VStanz ist wie folgt definiert:

$$V_{STanz} = \pi \times r^2 \times h$$

r ist der Radius des Stanzstücks, h die Höhe der ausgestanzten Schülpe.

Bezüglich der scheinbaren Dichte der Schülpe gilt hier analog die Beziehung

$$\text{scheinbare Dichte}_{\text{Schülpe}} = \text{Masse}_{\text{Stanz}} / \text{Volumen}_{\text{Stanz}}$$

Die Stanzmethode b) findet bevorzugt immer dann Anwendung, wenn die Durchsatzmethode a) aufgrund der Gerätegeometrie im Verfahrenschritt (ii) nicht anwendbar ist.

Die im Verfahrensschritt (ii) resultierende Schülpe ist weiterhin durch die Porosität charekterisierbar. Sie weist üblicherweise eine Porosität zwischen 0,16 und 0,45, bevorzugt zwischen 0,25 und 0,43, besonders bevorzugt zwischen 0,28 und 0,40 auf.

Der typische Durchsatz durch den Kompaktor beträgt üblicherweise 12-45 kg/h, bevorzugt 15 - 30 kg/h. Hierfür wird bevorzugt die oben genannte Spaltenbreite (insbesondere 2,5 bis 4,5 mm) und eine Walzenbreite von 100 mm verwendet.

Die Berechnung der Porosität erfolgt nach der Formel:

**Porosität Epsilon = (1-(wahre Dichte Ausgangsmaterial/scheinbare Dichte Schülpe)**

Bei dem Ausgangsmaterial handelt es sich um das in Verfahrensschritt (i) erhaltene Gemisch. Die wahre Dichte kann mit einem Gaspyknometer bestimmt werden. Vorzugsweise handelt es sich bei dem Gaspyknometer um ein Helium-Pykhometer, insbesondere wird das Gerät AccuPyc 1340 Helium Pyknometer des Herstellers Micromeritics, Deutschland, verwendet.

Beispiel für die Berechnung der Porosität:

| Wahre Dichte Ausgangsmaterial | 1.4 g/cm$^3$ | 1.6 g/cm$^3$ | 1.4 g/cm$^3$ | 1,4 g/cm$^3$ |
|---|---|---|---|---|
| Durchsatz | 15 kg/h | 15 kg/h | 45 kg/h | 15 kg/h |
| Spaltbreite | 3,24 mm | 3,24 mm | 3,24 mm | 5,00mm |
| Walzendrehzahl | 1/min | 1/min | 3/min | 1/min |
| Walzendurchmesser | 250 mm | 250 mm | 250 mm | 250 mm |
| Walzenbreite | 100 mm | 100 mm | 100 mm | 100mm |

Ergebnisse:

| | | | | |
|---|---|---|---|---|
| scheinbare Dichte | 0,982 g/cm$^3$ | 0,982 g/cm$^3$ | 0,982 g/cm$^3$ | 0,637 g/cm$^3$ |
| Porosität | 0.298 | 0,386 | 0,298 | 0,545 |

In Schritt (iii) des erfindungsgemäßen Verfahrens wird die Schülpe granuliert. Die Granulierung kann mit im Stand der Technik bekannten Verfahren erfolgen.

In einer bevorzugten Ausführungsform werden die Granulierungsbedingungen so gewählt, dass die resultierenden Teilchen (Granulate) eine volumenmittlere Teilchengröße (D50-Wert) von 20 bis 600 $\mu$m aufweisen, mehr bevorzugt von 50 bis 400 $\mu$m, noch mehr bevorzugt 80 bis 200 $\mu$m, insbesondere von 90 bis 130 $\mu$m. Der D90-Wert der resultierenden Teilchen (Granulate) liegt üblicherweise bei 500 bis 1300 $\mu$m, bevorzugt bei 800 bis 1200 $\mu$m.

In einer alternativen bevorzugten Ausführungsform beträgt der D50-Wert 30 $\mu$m bis 200 $\mu$m und/oder der D90-Wert 250 $\mu$m bis 1200 $\mu$m.

Weiterhin werden die Granulierbedingungen bevorzugt so gewählt, dass die resultierenden Granulate eine Schüttdichte von 0,3 bis 0,85 g/ml, mehr bevorzugt 0,4 bis 0,8 g/ml, insbesondere 0,5 bis 0,7 g/ml aufweisen. Der Hausner-Faktor liegt üblicherweise im Bereich von 1,02 bis 1,4, mehr bevorzugt von 1,04 bis 1,20 und insbesondere von 1,1 bis 1,25. Unter "Hausner-Faktor" wird hierbei das Verhältnis von Stampfdichte zu Schüttdichte verstanden.

Die volumenmittlere Teilchengröße (D50) der tablettierfertigen Endmischung liegt bevorzugt bei 20 - 300 $\mu$m, besonders bevorzugt bei 45 - 100 $\mu$m. Der D90-Wert dieser Teilchengrößenverteilung liegt bevorzugt bei 350 - 1500 $\mu$m, besonders bevorzugt bei 500 - 1000 $\mu$m.

In einer bevorzugten Ausführungsform erfolgt die Granulierung mittels Granulatorsieb, welches im Kompaktor integriert oder separat sein kann; oder in einer anderen Siebmühle. In diesem Fall beträgt die Maschenweite des Siebeinsatzes üblicherweise 0,1 bis 4 mm, bevorzugt 0,5 bis 2 mm, mehr bevorzugt 0,8 bis 2 mm, insbesondere 1,0 bis 1,5 mm.

Gegebenenfalls können die Moxifloxacin-Adhäsionsmittel-Teilchen eine nicht ausreichend rauhe Oberfläche aufweisen, so dass der vorstehend beschriebene Kompaktierungsschritt (ii) erschwert wird. Daher kann, je nach Oberflächen-beschaffenheit, der Kompaktierschritt (ii) und der Granulierschritt (iii) bei Bedarf wiederholt werden.

In einer weiteren Ausführungsform wird daher das erfindungsgemäße Verfahren derart angepasst, dass eine Mehrfachkompaktierung erfolgt, wobei das aus Schritt (iii) resultierende Granulat einmal oder mehrmals zur Kompaktierung (ii) rückgeführt wird.

Bevorzugt wird das Granulat aus Schritt (iii) 1 bis 5 mal rückgeführt, insbesondere 2 bis 3 mal.

Im Falle der Mehrfachkompaktierung erfolgt die Granulierung (iii) bevorzugt mittels eines sogenannten Frewitt Siebs. Es wird bevorzugt mit Siebdurchmessern von 50 bis 250 $\mu$m gesiebt.

Im Falle der Mehrfachkompaktierung ist es außerdem möglich, dass die vorstehend angegebenen Hilfsstoffmengen nur teilweise im Schritt (i) zugegeben werden, wobei die verbleibenden Teilmengen vor den weiteren Kompaktiervorgängen zugegeben werden.

Die vorstehend gemachten Ausführungen zu den Schritten (i) und (ii) finden hierbei nicht nur zur Herstellung der erfindungsgemäßen Tabletten, sondern auch zur Herstellung der erfindungsgemäßen Schülpe Anwendung. Die vorstehenden Ausführungen zu den Schritten (i) bis (iii) finden analog auch zur Herstellung der erfindungsgemäßen Granulate Anwendung.

**In Schritt (iv)** des erfindungsgemäßen Verfahrens werden die in Schritt (iii) erhaltenen Granulate zu Tabletten verpresst, d.h. es erfolgt eine Kompression zu Tabletten. Die Kompression kann mit im Stand der Technik bekannten Tablettiermaschinen erfolgen. Verfahrensschritt (iv) erfolgt bevorzugt in Abwesenheit von Lösungsmitteln, insbesondere organischen Lösungsmitteln, d.h. als Trockenkompression. Beispiele für geeignete Tablettiermaschinen sind Exzenterpressen oder Rundlaufpressen. Beispielsweise kann eine Fette 102i (Fette GmbH, Germany) verwendet werden. Im Falle von Rundlaufpressen und Exzenterpressen wird üblicherweise eine Presskraft von 2 bis 40 kN, bevorzugt von 2,5 bis 35 kN, angewandt. Mit Exzenterpressen sind aber auch Presskräfte bis 100 kN möglich.

In Schritt (iv) des erfindungsgemäßen Verfahrens können den Granulaten aus Schritt (iii) Hilfsstoffe zugegeben werden.

Beispiele für geeignete Hilfsstoffe sind beispielsweise Zusätze zur Verbesserung der Pulverfließfähigkeit (z.B. disperses Siliciumdioxid), Tablettenschmiermittel (z.B. Talk, Stearinsäure, Adipinsäure, Natriumstearylfumarat und/oder Magnesiumstearat) und Sprengmittel. Des Weiteren können auch die unter Schritt (i) erwähnten Adhäsionsmittel zugesetzt werden. Insbesondere werden im Schritt (iv) Fließreguliermittel und/oder Schmiermittel zugegeben. Sofern nicht bereits im Schritt (i) zugesetzt, erfolgt im Schritt (iv) bevorzugt auch ein Zusatz von Sprengmittel.

Als Sprengmittel werden im Allgemeinen Stoffe bezeichnet, die den Zerfall einer Darreichungsform, insbesondere einer Tablette, nach Einbringen in Wasser beschleunigen. Geeignete Sprengmittel sind z.B. organische Sprengmittel wie Carrageenan, Croscarmellose und Crospovidon.

Alternativ können alkalische Sprengmittel verwendet werden. Unter alkalischen Sprengmitteln sind Sprengmittel zu verstehen, die beim Lösen in Wasser einen pH-Wert von mehr als 7,0 erzeugen. Bevorzugt können anorganische alkalische Sprengmittel verwendet werden, insbesondere Salze von Alkali- und Erdalkalimetallen. Bevorzugt sind hier Natrium, Kalium, Magnesium und Calcium zu nennen. Als Anionen sind Carbonat, Hydrogencarbonat, Phosphat, Hydrogenphosphat und Dihydrogenphosphat bevorzugt. Beispiele sind Natriumhydrogencarbonat, Natriumhydrogenphosphat, Calciumhydrogencarbonat und dergleichen.

Sprengmittel werden üblicherweise in einer Menge von 0,5 bis 15 Gew.-%, bevorzugt 3 bis 7 Gew.-%, bezogen auf das Gesamtgewicht der Formulierung, verwendet.

Ein Beispiel für einen Zusatz zur Verbesserung der Pulverfließfähigkeit (Fließreguliermittel) ist disperses Siliciumdioxid, z.B. bekannt unter dem Handelsnamen Aerosil®. Bevorzugt wird Siliciumdioxid mit einer spezifischen Oberfläche von 50 bis 400 $m^2/g$, bestimmt nach Gasadsorption gemäß Ph. Eur., 6. Auflage, 2.9.26, verwendet. Fließregulierungsmittel haben üblicherweise die Aufgabe, sowohl die Reibung (Kohäsion) zwischen den einzelnen Pulverpartikeln bzw. Granulatkörnern als auch das Haften dieser an den Wandflächen der Pressvorrichtung zu vermindern.

Zusätze zur Verbesserung der Pulverfließfähigkeit werden üblicherweise in einer Menge von 0,1 bis 3 Gew.-%, bezogen auf das Gesamtgewicht der Formulierung, verwendet.

Ferner können Schmiermittel verwendet werden. Schmiermittel dienen im Allgemeinen zur Verringerung der Gleitreibung. Insbesondere soll die Gleitreibung vermindert werden, die beim Tablettieren einerseits zwischen den sich in der Matrizenbohrung auf und ab bewegenden Stempeln und der Matrizenwand sowie andererseits zwischen Tablettensteg und Matrizenwand besteht. Geeignete Schmiermittel stellen z.B. Stearinsäure, Adipinsäure, Natriumstearylfumarat und/oder Magnesiumstearat dar.

Schmiermittel werden üblicherweise in einer Menge von 0,1 bis 3 Gew.-%, bezogen auf das Gesamtgewicht der Formulierung, verwendet.

Die Menge an Hilfsstoffen, die im Schritt (iv) zugesetzt wird, hängt üblicherweise von der Art der herzustellenden Tablette ab und von der Menge an Hilfsstoffen, die bereits in den Schritten (i) oder (ii) zugesetzt wurde.

Das Verhältnis Wirkstoffe zu Hilfsstoffe wird bevorzugt so gewählt, dass die resultierende Tabletten

(a) 35 bis 85 Gew.-%, mehr bevorzugt 45 bis 75 Gew.-%, insbesondere 55 bis 65 Gew.-% Moxifloxacin oder dessen pharmazeutische verträgliche Salze, und

(b) 15 bis 65 Gew.-%, mehr bevorzugt 25 bis 55 Gew.-%, insbesondere 35 bis 45 Gew.-% pharmazeutisch verträgliche Hilfsstoffe, bezogen auf das Gesamtgewicht der unbefilmten Tablette, enthalten.

Diese Mengenangaben sind insbesondere bevorzugt, wenn mit dem erfindungsgemäßen Verfahren Tabletten hergestellt werden, die unzerkaut geschluckt werden.

Bei den durch das erfindungsgemäße Verfahren hergestellten Tabletten kann es sich also um Tabletten, die unzerkaut geschluckt werden (unbefilmt oder bevorzugt befilmt), handeln. Ebenfalls kann es sich um Kautabletten oder um Disperstabletten handeln. Unter "Disperstablette" wird hierbei eine Tablette zur Herstellung einer wässrigen Suspension zum Einnehmen verstanden.

Die Tablettierbedingungen werden im erfindungsgemäßen Verfahrens ferner bevorzugt so gewählt, dass die resultierenden Tabletten ein Verhältnis von Tablettenhöhe zu Gewicht von 0,005 bis 0,3 mm/mg, besonders bevorzugt 0,005 bis 0,01 mm/mg, aufweisen.

Ferner weisen die resultierenden Tabletten bevorzugt eine Bruchfestigkeit von 160 bis 400 N, besonders bevorzugt von 200 bis 350 N, insbesondere von 220 bis 270 N, auf. Die Bruchfestigkeit wird gemäß Ph.Eur.6, Ausgabe Grundwerk 2008. 2.9.8 bestimmt.

Zudem zeigen die resultierenden Tabletten bevorzugt eine Friabilität von kleiner 2 %, besonders bevorzugt von kleiner 1 %, insbesondere kleiner 0,5 %, auf. Die Friabilität wird gemäß Ph.Eur. 6.0, Abschnitt 2.9.7 bestimmt.

Schließlich weisen die erfindungsgemäßen Tabletten üblicherweise eine Gleichförmigkeit des Gehalts (*Content Uniformity*) von 95 bis 105 %, bevorzugt von 97 bis 103 %, insbesondere von 99 bis 101 % vom durchschnittlichen Gehalt auf. Die "Content Uniformity" wird gemäß Ph. Eur.6.0. Abschnitt 2.9.6. bestimmt.

Das Freisetzungsprofil der erfindungsgemäßen Tabletten weist im Falle einer IR-Formulierung gemäß USP-Methode (bevorzugt Methode "paddle") nach 10 Minuten üblicherweise einen freigesetzten Gehalt von mindestens 30 %, bevorzugt mindestens 60 %, insbesondere mindestens 80 %, auf.

Die vorstehenden Angaben zu Bruchfestigkeit, Friabilität, Content Uniformity und Freisetzungsprofil beziehen sich hierbei bevorzugt auf die unbefilmte Tablette.

In einer bevorzugten Ausführungsform wird im erfindungsgemäßen Verfahren keine Lactose oder zumindest nur geringe Mengen zugegeben. Trotz der Abwesenheit von Lactose wird die gewünschte Bruchfestigkeit erreicht. Gegenstand der Erfindung ist daher eine Tablette umfassend Moxifloxacin, enthaltend weniger als 10 Gew.-% Lactose, bevorzugt weniger als 5 Gew.-% Lactose, die insbesondere im Wesentlichen frei von Lactose ist, wobei die Tablette eine Bruchfestigkeit von 140 bis 400 N aufweist.

Im optionalen Schritt (v) des erfindungsgemäßen Verfahrens werden die Tabletten aus Schritt (iv) befilmt. Hierbei können die im Stand der Technik üblichen Verfahren zur Befilmung von Tabletten Anwendung finden.

Für die Befilmung werden bevorzugt makromolekulare Stoffe verwendet, beispielsweise modifizierte Cellulosen. Polymethacrylate, Polyvinylpyrrolidon, Polyvinylacetatphthalat, Zein und/oder Schellack.

Die Schichtdicke des Überzugs beträgt bevorzugt 10 bis 100 $\mu$m, mehr bevorzugt 15 bis 50 $\mu$m.

Das erfindungsgemäße Verfahren ist insbesondere zur Herstellung von Tabletten geeignet, die eine große Menge an Moxifloxacin oder pharmazeutisch verträgliche Salze davon enthalten. In einer bevorzugten Ausführungsform enthalten die erfindungsgemäßen Tabletten 100 bis 1000 mg, besonders bevorzugt 200 bis 800 mg, insbesondere 200 bis 600 mg Moxifloxacin oder pharmazeutisch verträgliche Salze davon.

Es hat sich gezeigt, dass das erfindungsgemäße Verfahren mit folgenden Stoffmengen bevorzugt durchgeführt werden kann:

200 bis 600 mg Moxifloxacin, bevorzugt 380 bis 450 mg Moxifloxacin, wobei Moxifloxacin bevorzugt als freie Base oder als Hydrochlorid verwendet wird;

100 bis 300 mg Adhäsionsmittel, bevorzugt 180 bis 250 mg Adhäsionsmittel, wobei als Adhäsionsmittel bevorzugt mikrokristalline Cellulose verwendet wird;

20 bis 50 mg, bevorzugt 28 bis 38 mg Sprengmittel, wobei als Sprengmittel bevorzugt Croscarmellose Natrium verwendet wird;

0 bis 10 mg, bevorzugt 3 bis 8 mg Fließregulierungsmittel, wobei als Fließregulierungsmittel bevorzugt disperses Siliciumdioxid (Aerosil®) verwendet wird; und

0 bis 20 mg, bevorzugt 5 bis 15 mg Schmiermittel, wobei als Schmiermittel bevorzugt Magnesiumstearat verwendet wird.

Das erfindungsgemäße Verfahren wird also bevorzugt mit oben genannten Einsatzstoffen durchgeführt. Gegenstand der Erfindung sind somit auch Tabletten, die nach dem erfindungsgemäßen Verfahren erhältlich sind und die vorstehend genannte Formulierung enthalten.

Gegenstand der Erfindung ist, wie vorstehend erläutert, also nicht nur das erfindungsgemäße Verfahren, sondern auch die mit diesem Verfahren hergestellten Tabletten. Es wurde gefunden, dass die mit diesem Verfahren hergestellten Tabletten eine bimodale Porengrößenverteilung aufweisen können. Offenbart werden somit Tabletten, enthaltend Moxifloxacin oder pharmazeutisch verträgliche Salze davon sowie gegebenenfalls pharmazeutisch verträgliche Hilfsstoffe, wobei die Tabletten eine bimodale Porengrößenverteilung aufweisen.

Die erfindungsgemäße Tablette entsteht, wenn das Granulat aus Verfahrensschritt (iii) komprimiert wird. Dieses Komprimat besteht aus Feststoff und Poren. Die Porenstruktur kann durch Bestimmung der Porengrößenverteilung näher charakterisiert werden.

Die Porengrößenverteilung wurde mittels Quecksilberporosimetrie bestimmt. Quecksilberporosimetriemessungen erfolgten mit dem Porosimeter "Poresizer" der Firma Micromeritics, Norcross, USA. Die Porengrößen wurden hierbei unter Annahme einer Oberflächenspannung von Quecksilber von 485 mN/m berechnet. Aus dem kumulativen Porenvolumen wurde die Porengrößenverteilung als Summenverteilung beziehungsweise Anteil der Porenfraktionen in Prozent errechnet. Der durchschnittliche Porendurchmesser (4V/A) wurde aus dem gesamten spezifischen Quecksilberintrusionsvolumen ($Vges_{int}$) und der gesamten Porenfläche ($Agesp_{por}$) nach folgender Gleichung ermittelt.

$$4V/A = \frac{4 \cdot Vges_{int}\ [ml/g]}{Ages_{por}\ [m^2/g]}$$

Unter "bimodaler Porengrößenverteilung" wird verstanden, dass die Porengrößenverteilung zwei Maxima aufweist.

Die Erfindung soll anhand der nachfolgenden Beispiele veranschaulicht werden.

## BEISPIELE

### Erfindungsgemäße Beispiele 1 bis 3

Eine Mischung aus Moxifloxacin-Hydrochlorid und Adhäsionsmittel wurde hergestellt, indem Moxifloxacin und Adhäsionsmittel sowie Croscarmellose Na mit einem Freifallmischer für 10 min intensiv gemischt wurden. Anschließend wurde diese Mischung auf einem pharmagerechten Walzenkompaktor mit einer Spaltbreite von 3,5 mm und über ein Brechsieb mit Maschenweite 1,25 mm gebrochen. Das erhaltene gebrochenen Kompaktat (= Granulat) wurde mit hochdispersem Siliciumdioxid nach dem Sieben gemischt (Freifallmischer Typ Rhönrad) und mit Magnesiumstearat endgemischt (Freifallmischer Typ Rhönrad). Nach dem Verpressen auf einer Hochleistungs-Rundläufertablettenpresse zu Tabletten vorgegebener Größe wurden die für die Arzneiform üblichen Inprozesskontrollen durchgeführt.

Die eingesetzten Mengen ergeben sich aus Tabelle 1.

### Erfindungsgemäße Beispiele 1a bis 3a

Die Tablettenkerne gemäß den Beispielen 1 bis 5 wurden befilmt. Dazu wurde Hypromellose (Pharmacoat 603) und Polyethylenglykol 6000 mit Wasser angesetzt, und nach dem Auflösen mit separat angesetzter Suspension aus Titandioxid, Eisenoxid in Wasser vereinigt. Die Tablettenkerne wurden mit der resultierenden Suspension im Lochtrommelcoater überzogen. Die eingesetzten Mengen ergeben sich aus Tabelle 1.

Alternativ konnten auch Fertiglacke sowie unterschiedliche Anteile an Hypromellose (gewichtsmittleres Molekulargewicht 10.000 - 150.000), Polyethylenglykol (gewichtsmittleres Molekulargewicht 200 bis 8.000), Titandioxid und Farbpigmenten verwendet werden.

### Erfindungsgemäße Beispiele 4, 4a, 5 und 5a

Analog zu den Beispielen 1-3 und 1a-3a werden die Beispiele 4-5 und 4a-5a hergestellt, wobei anstelle des Hydrochlorid-Salzes Moxifloxacin in Form der freien Base eingesetzt wird.

Die eingesetzten Mengen ergeben sich aus Tabelle 2.

### **Vergleichsbeispiel** 6

Es wurde ein Tablettenkern, wie in Beispiel 6 von EP 1 128 831 B1 beschrieben, mittels Nassgranulierung hergestellt. Die eingesetzten Mengen ergeben sich aus Tabelle 3.

### Vergleich der physikalischen Eigenschaften

Die physikalischen Eigenschaften der resultierenden Tabletten werden in Tabelle 4 verglichen.

Es zeigt sich, dass die mittels (Trocken)-Kompaktierung hergestellten Tabletten (sowohl befilmt als auch unbefilmt) vorteilhafte Eigenschaften im Hinblick auf die Bruchfestigkeit und die Friabilität zeigen. Ebenfalls sind die erfindungemäßen Tabletten besser lackierbar, es kommt zu weniger Abplatzern.

Ferner zeigt sich, dass lactosefreie Formulierungen eine bessere Gleichförmigkeit des Gehalts (content *uniformity*) zeigen.

Zusammenfassend ist festzustellen, dass mit dem erfindungsgemäßen Verfahren die physikalischen Eigenschaften der resultierenden Tabletten positiv beeinflusst werden konnten. Weiterhin ist das erfindungsgemäße Verfahren unter dem Aspekt der Senkung des Energieaufwandes vorteilhaft, der bei dem Verfahren des Standes der Technik nicht optimal ist, weil die Feuchte in Form von Granulierlösung erst eingebracht und dann in einem aufwendigen Trockenprozess wieder entfernt werden muss.

**Tabelle 1**

| | **Beispiel 1** | | **Beispiel 2** | | **Beispiel 3** | |
|---|---|---|---|---|---|---|
| **Kern** | mg | Gew.-% | mg | Gew.-% | mg | Gew.-% |
| Moxifloxacin-HCl | 436,80 | 64,35 | 436,80 | 64,35 | 436,80 | 64,35 |
| MCC (Mikrokr. Cellulose) | **195,00** | 28,73 | **128,00** | 18,86 | **136,00** | 20,04 |
| Lactose-Monohydrat | **0,00** | 0,00 | **0,00** | 0,00 | **68,00** | 10,02 |
| Mannit | 0,00 | 0,00 | **67,00** | 9,87 | 0,00 | 0,00 |
| Croscarmellose Na | 32,00 | 4,71 | 32,00 | 4,71 | 32,00 | 4,71 |
| Mg-Stearat (E572) | 10,00 | 1,47 | 10,00 | 1,47 | 6,00 | 0,88 |
| Aerosil | 5.00 | 0,74 | 5,00 | 0,74 | 0,00 | 0,00 |
| | **678,80** | **100,00** | **678,80** | **100,00** | **678,80** | **100,00** |
| | | | | | | |
| **Film** | **Beispiel 1a** | | **Beispiel 2a** | | **Beispiel 3a** | |
| Hypromellose | 10,80 | | 10,80 | | 10,80 | |
| Titandioxid | 3,24 | | 3,24 | | 3,24 | |
| Polyethylenglykol | 3,60 | | 3,60 | | 3,60 | |
| Eisenoxid, rot | 0,36 | | 0,36 | | 0,36 | |
| | **18,00** | | **18,00** | | **18,00** | |
| | | | | | | |
| **Filmtablette (FT)** | 696,80 | | 696,80 | | 696,80 | |
| | | | | | | |
| **Herstellverfahren** | Trockengranulation | | Trockengranulation | | Trockengranulation | |

**Tabelle 2**

| | **Beispiel 4** | | **Beispiel 5** | |
|---|---|---|---|---|
| **1. Kern** | mg | Gew.-% | mg | Gew.-% |
| Moxifloxacin (freie Base) | 400,00 | 58,93 | 400,00 | 58,93 |
| MCC (Mikrokr. Cellulose) | **231,80** | 34,15 | **230,80** | 34,00 |
| Croscarmellose Na | 32,00 | 4,71 | **5,00** | 0,74 |
| Aerosil | 5,00 | 0,74 | 5,00 | 0,74 |
| Kollidon VA 64 | 0,00 | 0,00 | **28,00** | 4,12 |
| Mg-Stearat (E572) | 10,00 | 1,47 | 10,00 | 1,47 |
| | **678,80** | **100,00** | **678,80** | **100,00** |
| | | | | |
| **Film** | **Beispiel 4a** | | **Beispiel 5a** | |
| Hypromellose | 10,80 | | 10,80 | |
| Titandioxid | 3,24 | | 3,24 | |
| Polyethyleneglykol | 3,60 | | 3,60 | |
| Eisenoxid, rot | 0,36 | | 0,36 | |
| | **18,00** | | **18.00** | |
| | | | | |
| **Filmtablette (FT)** | 696,80 | | 696,80 | |
| | | | | |
| **Herstellverfahren** | Trockengranulatton | | Trockengranulation | |

**Tabelle 3**

| **Kern** | **Beispiel 6** (Vergleich) | Gew.-% |
|---|---|---|
| Moxifloxacin-HCl | 436,80 | 64,35 |
| MCC (E460 (I)) | **136,00** | 20,04 |
| Lactose-Monohydrat | **68,00** | 10,02 |
| Mannit | **0,00** | 0,00 |
| Crosscarmellose Na | 32,00 | 4,71 |
| Mg-Stearat (E572) | 6,00 | 0,88 |
| Aerosil | 0,00 | 0,00 |
| | **678.80** | **100,00** |
| | | |
| **Film** | **keine Filmtabletten hergestellt** | |
| | | |
| **Herstellverfahren** | wässrige Granulation | |

**Tabelle 4**

| | **Beispiel** 6 (Vergleich) | **Beispiel** 1 | **Beispiel 2** | **Beispiel** 3 | **Beispiel** 4 | **Beispiel 5** |
|---|---|---|---|---|---|---|
| **Presskraft [kN]**<br>VPK<br>HPK<br>srel [%] | <br>14<br>23<br>4,3 | <br>12<br>23<br>5,5 | <br>12<br>**23**<br>**4,2-6,8** | <br>12<br>**23**<br>**8,6** | <br>8<br>15<br>7,22 | <br>8<br>15<br>4,78 |
| **BFK Kerne [N] /srel. [%]** | 152,66 / 7,42 | 250,72 / 7,17 | 230,43 / 5,94 | 235,6 / 5,36 | 188 / 7,32 | 200 / 5,63 |
| **Abrieb Kerne [%]** | gedeckelt; >1% | 0,26 | 0,25 | 0,31 | 0,55 | 0,41 |
| **Abplatzer [%]** | 1 | 0 | 0 | 0 | 0 | 0 |
| **BFK FT [N] / srel. [%]** | nicht lacklerfähig | 313,13 / 6,13 | 285,6 / 6,77 | 290,03 / 8,95 | 271,73 | 284 |
| **Zerfall FT [min]** | keine FT hergestellt | 1'43" | 2'29" | 2'25" | 1'25" | 4'07" |
| BFK = Bruchfestigkeit<br>VPK = Vorpresskraft<br>HPK = Hauptpresskraft<br>srel = relative Standardabweichung<br>FT = Filmtablette<br>Kern = unbefilmte Tablette | | | | | | |

**Patentansprüche**

1. Verfahren zur Herstellung von Tabletten enthaltend Moxifloxacin, umfassend die Schritte

   (i) Bereitstellen von Moxifloxacin oder pharmazeutisch verträglicher Salze davon im Gemisch mit einem Adhäsionsmittel;
   (ii) Kompaktierung zu einer Schülpe; wobei die Kompaktierung in einem Walzengranulator durchgeführt wird und wobei die Spaltbreite des Walzengranulators 2 bis 4 mm und wobei die Walzkraft 2 bis 30 kN/cm beträgt.
   (iii) Granulierung der Schülpe; und
   (iv) Kompression der resultierenden Granulate zu Tabletten, gegebenenfalls unter Zusatz weiterer pharmazeutischer Hilfsstoffe.

2. Verfahren nach Anspruch 1, wobei die Kompaktierungsbedingungen im Schritt (ii) so gewählt werden, dass die Schülpe eine scheinbare Dichte von 0,86 bis 1,38 g/$cm^3$ aufweist.

3. Verfahren nach Anspruch 1 oder 2, wobei die Granulierungsbedingungen im Schritt (iii) so gewählt werden, dass die resultierenden Teilchen eine volumenmittlere Teilchengröße D50 von 80 $\mu$m bis 500 $\mu$m und einen D90-Wert von 800 bis 1200 $\mu$m aufweisen.

4. Verfahren nach einem der Ansprüche 1 bis 3, wobei das Adhäsionsmittel aus mikrokristalliner Cellulose oder einem Gemisch aus mikrokristalliner Cellulose sowie Mannitol und/oder Sorbitol besteht.

5. Verfahren nach einem der Ansprüche 1 bis 4, **dadurch gekennzeichnet, dass** in Schritt (i)

   (a) 50 bis 85 Gew.-% Moxifloxacin oder dessen pharmazeutisch verträgliche Salze und
   (b) 15 bis 50 Gew.-% Adhäsionsmittel vermischt werden, bezogen auf das Gesamtgewicht des Gemisches aus Schritt (i).

6. Verfahren nach einem der Ansprüche 1 bis 5, wobei im Verfahrensschritt (iv) weitere Hilfsstoffe, insbesondere Sprengmittel, Fließreguliermittel und/oder Schmiermittel zugegeben werden.

**7.** Verfahren nach einem der Ansprüche 1 bis 6, wobei die Tablette in einem Schritt (v) zusätzlich befilmt wird.

**8.** Tablette enthaltend Moxifloxacin oder pharmazeutisch verträgliche Salze davon, erhältlich nach einem Verfahren gemäß einem der Ansprüche 1 bis 7.

**9.** Tablette nach Anspruch 8, **dadurch gekennzeichnet, dass** die Tablette im Wesentlichen frei von Laktose ist und eine Bruchfestigkeit von 160 bis 300 N aufweist.

**10.** Tablette nach Anspruch 8 oder 9, wobei die Tablette eine Friabilität von kleiner 1 % und eine Gleichförmigkeit des Gehalts von 95 bis 105 % aufweist.

**11.** Schülpe enthaltend Moxifloxacin, erhältlich durch ein Verfahren umfassend die Schritte

(i) Bereitstellen von Moxifloxacin oder pharmazeutisch verträglicher Salze davon im Gemisch mit einem Adhäsionsmittel; und
(ii) Kompaktierung zu einer Schülpe; wobei die Kompaktierung in einem Walzengranulator durchgeführt wird und wobei die Spaltbreite des Walzengranulators 2 bis 4 mm und wobei die Walzkraft 2 bis 30 kN/cm beträgt.

**12.** Granulat, insbesondere zur Befüllung von Sachets oder Kapseln, enthaltend Moxifloxacin, erhältlich durch ein Verfahren umfassend die Schritte

(i) Bereitstellen von Moxifloxacin oder pharmazeutisch verträglicher Salze davon im Gemisch mit einem Adhäsionsmittel;
(ii) Kompaktierung zu einer Schülpe; wobei die Kompaktierung in einem Walzengranulator durchgeführt wird und wobei die Spaltbreite des Walzengranulators 2 bis 4 mm, und wobei die Walzkraft 2 bis 30 kN/cm beträgt, und
(iii) Granulierung der Schülpe.

## Claims

**1.** Process for the preparation of tablets containing moxifloxacin, comprising the steps of

(i) providing moxifloxacin or pharmaceutically acceptable salts thereof mixed with an adhesive agent;
(ii) compacting it into a slug; wherein the compacting is carried out in a roll granulator and wherein the gap width of the roll granulator is 2 to 4 mm and the rolling force is 2 to 30 kN/cm
(iii) granulating the slug; and
(iv) compressing the resulting granules into tablets, optionally with the addition of further pharmaceutical excipients.

**2.** Process according to claim 1, wherein the compacting conditions in step (ii) are selected such that the slug has an apparent density of 0.86 to 1.38 g/cm$^3$.

**3.** Process according to claim 1 or 2, wherein the granulation conditions in step (iii) are selected such that the resulting particles have a volume-average particle size D50 of 80 $\mu$m to 500 $\mu$m and a D90 value of 800 to 1200 $\mu$m.

**4.** Process according to any one of claims 1 to 3, wherein the adhesive agent consists of microcrystalline cellulose or a mixture of microcrystalline cellulose and mannitol and/or sorbitol.

**5.** Process according to any one of claims 1 to 4, **characterised in that** in step (i)

(a) 50 to 85 % by weight moxifloxacin or its pharmaceutically acceptable salts; and
(b) 15 to 50 % by weight adhesive agent are mixed, based on the total weight of the mixture from step (i).

**6.** Process according to any one of claims 1 to 5, wherein in process step (iv) further excipients, especially disintegrants, flow-regulating agents and/or lubricants are added.

**7.** Process according to any one of claims 1 to 6, wherein the tablet is additionally film-coated in a step (v).

8. Tablet comprising moxifloxacin or pharmaceutically acceptable salts thereof, obtainable by a process according to any one of claims 1 to 7.

9. Tablet comprising moxifloxacin, **characterised in that** the tablet is substantially free of lactose and has a breaking strength of 160 to 300 N.

10. Tablet according to claim 8 or 9, wherein the tablet has a friability of less than 1 % and a content uniformity of 95 to 105 %.

11. Slug comprising moxifloxacin, obtainable by a process comprising the steps of

(i) providing moxifloxacin or pharmaceutically acceptable salts thereof mixed with an adhesive agent; and
(ii) compacting the mixture into a slug; wherein the compacting is carried out in a roll granulator and wherein the gap width of the roll granulator is 2 mm to 4 mm and a rolling force is 2 kN/cm to 30 kN/cm.

12. Granulate, especially for filling sachets or capsules, comprising moxifloxacin, obtainable by a process comprising the steps of

(i) providing moxifloxacin or pharmaceutically acceptable salts thereof mixed with an adhesive agent; and
(ii) compacting the mixture into a slug; wherein the compacting is carried out in a roll granulator and wherein the gap width of the roll granulator is 2 mm to 4 mm and a rolling force is 2 kN/cm to 30 kN/cm,
and
(iii) granulating the slug.

**Revendications**

1. Procédé de fabrication de comprimés contenant de la moxifloxacine, comprenant les étapes consistant à

(i) se procurer de la moxifloxacine ou ses sels pharmaceutiquement compatibles en mélange avec un promoteur d'adhérence ;
(ii) les compacter en une croûte, le compactage étant réalisé dans un granulateur à rouleaux et la largeur d'interstice du granulateur à rouleaux se situant entre 2 et 4 mm et la force de laminage entre 2 et 30 kN/cm.
(iii) granulation de la croûte ; et
(iv) compression des granulés en résultant en comprimés, éventuellement en y ajoutant d'autres excipients pharmaceutiques.

2. Procédé selon la revendication 1, dans lequel les conditions de compactage sont sélectionnées dans l'étape (ii) de manière à ce que la croûte présente une densité apparente de 0,86 à 1,38 g/$cm^3$.

3. Procédé selon la revendication 1 ou 2, dans lequel les conditions de granulation sont sélectionnées dans l'étape (iii) de manière à ce que les particules correspondantes présentent une taille de particules d'un volume moyen D50 de 80 $\mu$m à 500 $\mu$m et un indice D90 de 800 à 1200 $\mu$m.

4. Procédé selon une des revendications 1 à 3, dans lequel le promoteur d'adhérence est composé de cellulose microcristalline ou d'un mélange de cellulose microcristalline et de mannitol et/ou de sorbitol.

5. Procédé selon une des revendications 1 à 4, **caractérisé en ce que**, dans l'étape (i),

(a) 50 à 85 % en poids de moxifloxacine ou ses sels pharmaceutiques compatibles et
(b) 15 à 50 % en poids de promoteur d'adhérence sont mélangés, par rapport au poids total du mélange de l'étape (i).

6. Procédé selon une des revendications 1 à 5, dans lequel, dans l'étape (iv), d'autres excipients, en particulier des agents d'éclatement, régulateurs d'écoulement et/ou lubrifiants sont ajoutés.

7. Procédé selon une des revendications 1 à 6, dans lequel le comprimé est en plus recouvert d'un film dans une étape (v).

**8.** Comprimé contenant de la moxifloxacine ou ses sels pharmaceutiques compatibles, pouvant être obtenu suivant un procédé selon une des revendications 1 à 7.

**9.** Comprimé contenant de la moxifloxacine, **caractérisé en ce que** le comprimé est sensiblement exempt de lactose et présente une résistance à la rupture de 160 à 300 N.

**10.** Comprimé selon la revendication 8 ou 9, **caractérisé en ce que** le comprimé présente une friabilité inférieure à 1 % et une uniformité de contenu de 95 à 105 %.

**11.** Croûte contenant de la moxifloxacine, pouvant être obtenue par un procédé comprenant les étapes consistant à

(i) se procurer de la moxifloxacine ou ses sels pharmaceutiquement compatibles en mélange avec un promoteur d'adhérence ;
(ii) les compacter en une croûte, le compactage étant réalisé dans un granulateur à rouleaux et la largeur d'interstice du granulateur à rouleaux se situant entre 2 et 4 mm et la force de laminage entre 2 et 30 kN/cm.

**12.** Granulés, destinés en particulier à remplir des sachets ou gélules, contenant de la moxifloxacine pouvant être obtenue par un procédé comprenant les étapes consistant à

(i) se procurer de la moxifloxacine ou ses sels pharmaceutiquement compatibles en mélange avec un promoteur d'adhérence ;
(ii) les compacter en une croûte, le compactage étant réalisé dans un granulateur à rouleaux et la largeur d'interstice du granulateur à rouleaux se situant entre 2 et 4 mm et la force de laminage entre 2 et 30 kN/cm et
(iii) granulation de la croûte.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0350733 A2
- EP 0757990 B1
- DE 4200414 A1
- EP 0780390 A1
- EP 1017392 B1
- WO 200520998 A1
- WO 2007128349 A1
- EP 1128831 A
- EP 1128831 B1